Europäisches Patentamt
European Patent Office
Office européen des brevets

(11) Publication number: **0 225 011 A1**

(12) # EUROPEAN PATENT APPLICATION

(21) Application number: 86307436.5

(22) Date of filing: 29.09.86

(51) Int.Cl.⁴: C 07 C 103/58
C 07 C 102/00, C 07 C 59/68
C 07 D 317/28, C 07 F 9/44
A 01 N 39/02, A 01 N 43/00

(30) Priority: 30.09.85 GB 8524000

(43) Date of publication of application: 10.06.87 Bulletin 87/24

(84) Designated Contracting States: AT BE CH DE FR GB IT LI LU NL SE

(71) Applicant: THE WELLCOME FOUNDATION LIMITED
183-193 Euston Road
London NW1 2BP(GB)

(72) Inventor: Blade, Robert John
The Wellcome Research Laboratories Berkhamsted
Hertfordshire(GB)

(74) Representative: Bassett, Richard Simon et al,
Group Patents & Agreements The Wellcome Foundation Limited Langley Court
Beckenham Kent, BR3 3BS(GB)

(54) **Pesticidal (1'-naphthyloxy)-alkene (or -alkadiene)-carboxylic-acid amides.**

(57) Compounds of Formula (I):

(I) $Ar\text{-}O\text{-}(CH_2)_m\ (CH{=}CH)_a\ CONR^1R^2$

wherein Ar is a group 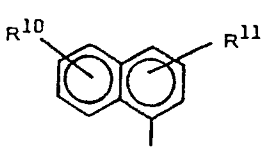

in which each $R^{10}$ and $R^{11}$ is independently one or more of hydrogen, alkyl, haloalkyl, halo, alkoxy or haloalkoxy

a = 1 or 2

m = 1 to 14

and $R^1$ and $R^2$ are independently selected from hydrogen, $C_{1-6}$ alkyl and $C_{3-6}$ cycloalkyl (either of which may be substituted by halo, $C_{2-6}$ alkenyl, $C_{1-6}$ alkyl, $C_{3-6}$ cycloalkyl, $\alpha C_{3-6}$ heterocycle wherein the heteroatoms are one or more oxygen atoms, $C_{1-6}$ alkoxy or $C_{2-6}$ alkynyl) are novel and have been found to have activity against insect and acarine pests.

EP 0 225 011 A1

TITLE MODIFIED
see front page

## Pesticidal Compounds

This invention relates to pesticidal compounds.

EP-A-143 593 (N.R.D.C., published 5th June 1985) discloses a wide range of lipid amide insecticides having an aryl or aryloxy group w to a diene-amide group. The aryloxy group may, amongst many other possibilities, be naphthyloxy, but it is stated that it is preferred for fused aromatic rings (including naphthyl) to be joined to the lipid chain at the 2-position relative to the ring system, and only 2-naphthyloxy compounds are exemplified. It has now surprisingly been found that 1-naphthyloxy compounds are in fact generally superior to 2-naphthyloxy compounds.

Accordingly the invention provides compounds of Formula (1)

(I) $Ar\text{-}O\text{-}(CH_2)_m\ (CH{=}CH)_a\ CONR^1R^2$

wherein Ar is a group

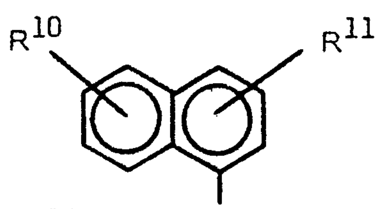

in which each of $R^{10}$ and $R^{11}$ is independently one or more of hydrogen, $C_{1-6}$ alkyl, halo $C_{1-6}$ alkyl, halo, $C_{1-6}$ alkoxy or halo $C_{1-6}$ alkoxy

a = 1 or 2
m = 1 to 14

and $R^1$ and $R^2$ are independently selected from hydrogen, $C_{1-6}$ alkyl and $C_{3-6}$ cycloalkyl (either of which may be substituted by halo, $C_{2-6}$ alkenyl, $C_{1-6}$ alkyl $C_{3-6}$ cycloalkyl, a $C_{3-6}$ heterocycle having one or more oxygens as the heteroatoms, $C_{1-6}$ alkoxy or $C_{2-6}$ alkynyl).

Preferably a is 2. Conveniently, the or each double bond conjugated to the amide carbonyl is in the E configuration.

Preferably, m is less than 8, most preferably 4 to 7. Suitably, m is an even number, most preferably 4 or 6.

Preferably $R^1$ is hydrogen. $R^2$ is suitably alkyl, alkenyl, alkoxy-alkyl or dioxalanyl-alkyl, $C_{1-5}$ alkyl being preferred. Isobutyl, 2,2-dimethylpropyl and 1,2-dimethyl propyl are particularly preferable.

$R^{10}$ and $R^{11}$ are preferably halo or trifluoromethyl. Suitably, the naphthyl nucleus is unsubstituted or monosubstituted, i.e. at least one of $R^{10}$ and $R^{11}$ is hydrogen. Conveniently, any substitution is in the 4, 5 or 7 position, preferably 5 or 7.

A particularly preferred compound is (2E,4E) N-isobutyl 9-(7-fluoro-1-naphthyloxy) nona-2,4-dienamide.

Compounds of Formula (1) may be prepared in any of the following ways:-

(a) by amidation of the corresponding acid or acid derivative, ie by reaction of a compound of Formula (II) with a compound of Formula (III) or derivative thereof

(II) $Ar\ O(CH_2)_m\ (CH{=}CH)_a COZ^1$

(III) $HNR^1R^2$

wherein $Z^1$ is hydroxyl, halo, or a phosphoroimidate ester group (-P(→O)(OAryl)NH aryl) and the other variables are as defined above.

(b) by reaction of a compound of Formula (IV) with a compound of Formula (V) or (VI)

(IV) $ArO(CH_2)_m(CH{=}CH)_pCHO$

(V) $(Z'')_3P{=}CH\ (CH{=}CH)_q\ C(O)NR^1R^2$

(VI) $(Z'')_2P(O){=}CH(CH{=}CH)_qC(O)NR^1R^2$

wherein Z" is alkyl, alkoxy (preferably ethoxy) or aryl (preferably phenyl), one of p and q is 0 and the other is 0 or 1. The locations of the aldehyde and the phosphorus-containing groups, $(Z'')_3P$ and $(Z'')_2P(O)$, may be swapped to give an exactly analogous reaction;

(c) by oxidation and elimination reactions on a compound of Formula (VII) or (VIII):

(VII) $Ar\text{-}O\text{-}(CH_2)_m(CH{=}CH)_{a-1}\ C(X)HC(Y)HC(O)NR^1R^2$

(VIII) $Ar\text{-}O(CH_2)_mC(X)H\ C(Y)H(CH{=}CH)_{a-1}\ C(O)NR^1R^2$

wherein one of X and Y is hydrogen and the other is a group Q(→O)L, Q is sulphur or selenium and L is a suitable group such as lower alkyl (preferably methyl) or aryl (preferably phenyl);

(d) by reaction of a compound of Formula (IX) with a compound of Formula (X):-

(IX) $Ar\text{-}O(CH_2)_mCH_2\text{-}Hal$

(X) $HC{\equiv}CH(CH{=}CH)_{a-1}C(O)NR^1R^2$

where Hal is a halogen atom, followed by reduction of the triple bond;

(e) by reacting a compound of Formula (XI) with a compound of Formula (XII):

(XI) $Ar\text{-}O(CH_2)_m\ CH{=}CH\text{-}M$

(XII) $Hal\text{-}(CH{=}CH)_{a-1}C(O)NR^1R^2$

wherein Hal is halide eg. bromide or iodide and M is a metal atom or metal group, for example comprising zirconium, aluminium or zinc, e.g. a bis-(cyclopentadienyl) zirconium chloride group; or

(f) by reduction of the triple bond in a compound of Formula (A) or (B):

(A) $ArO\ (CH_2)_m\ C{\equiv}C\text{-}\ (CH{=}CH)_{a-1}\text{-}CO\ NR^1R^2$

(B) $ArO\ (CH_2)_m\ (CH{=}CH)_{a-1}\text{-}C{\equiv}C\text{-}\ CO\ NR^1R^2$

for example by hydrogenation.

Process (a) is normally carried out in an aprotic solvent, such as ether, dichloromethane or benzene, optionally in the presence of a tertiary amine, such as

triethylamine, but in the absence of water. If the compound of Formula (II) is an acid halide, for example acid chloride, then it may be formed from the corresponding acid by reaction with a suitable reagent such as oxalyl chloride or thionyl chloride. When Z' is a phosphoroimidate group then this is suitably formed from (PhO)P(→O)NHPh Cl. The acid, or the acid function in the compound of Formula (II), may be prepared by hydrolysis of an ester, the ester being prepared by a conventional Wittig or Wadsworth-Emmons reaction, using for example an aldehyde and ethoxycarbonylmethylene triphenylphosphorane or the anion from triethylphosphonocrotonate. This latter reaction may result in an isomeric mixture, for example a (2,4)-hexadienoate and (3,5)-hexadienoate; such a mixture may be reacted as above, and the resulting mixture of amides separated by chromatography or other convenient techniques.

Alternatively, the ester referred to above may be derived by rearrangement and elimination on a compound of Formula (XIII):

(XIII) $Ar\text{-}O(CH_2)_{m+2}CH{=}C(S(\rightarrow O)R^3)COOR^4$

wherein $R^3$ is any suitable group, such as phenyl, and $R^4$ is alkyl.

The compound of Formula (XIII) may be obtained by reaction of a compound of Formula (XIV) with a compound of Formula (XV):

(XIV) $Ar\text{-}O(CH_2)_{m+2}\,CHO$

(XV) $R^3\,S(\rightarrow O)\,CH_2\,COOR^4$

A further route is for the ester referred to above to be prepared by rearrangement and elimination on a compound of Formula (XVI):

(XVI) $Ar\text{-}O(CH_2)_m\text{-}A^1\text{-}A^2\text{-}A^3\text{-}COOR^4$

wherein $R^4$ is as defined above, one of $A^1$, $A^2$ and $A^3$ is $(CH{=}CH)_{a-1}$, another of $A^1$, $A^2$ and $A^3$ is $-CH_2-$, the third of $A^1$, $A^2$ and $A^3$ is $-CH(OR^5)-$, $R^5$ being H or acyl such as acetyl, and the said $-CH_2-$ and $-CH(OR^5)-$ groups are adjacent one another. The reaction is preferably carried out in an aromatic solvent,

conveniently in the presence of a molybdenum catalyst and a base, such as bis-trimethylsilylacetamide .

Intermediates of Formula (XVI) may be obtained by reaction of a suitable aldehyde with a suitable sulphinyl compound, followed by rearrangement and acylation.

The reaction is carried out in a suitable solvent such as acetonitrile with a base such as piperidine.

Process (b) is carried out in a dry solvent, for example tetrahydrofuran, optionally in the presence of a base, and preferably in the absence of oxygen, e.g. under a nitrogen atmosphere, at a low temperature. The Wittig-type reagent may be obtained with lithium diisopropylamide.

The intermediates of Formulae (III) - (XIV) may also be prepared by standard methods. For example, the compounds of Formulae (V) and (VI) may be prepared by the reaction of an appropriate phosphine, phosphonate or phosphite with an w-halo amide. Compounds of Formula (IV) may be prepared by hydrolysis of a ketal ring or oxidation of an alcohol.

The carbonyl-containing compounds of Formula (IV) may be prepared by oxidation of the corresponding alcohol, for example using pyridinium chlorochromate or oxalyl chloride/DMSO.

The alcohols of Formula (XVII) may be prepared by reaction of compounds of Formula (XIX) with compounds of Formula (XX) by standard routes:

(XVII) $ArO(CH_2)_{m+1}OH$

(XIX) ArOH

(XX) $Y-(CH_2)_{m+1}OP$

where Y = halogen, preferably bromine or iodine, hydroxy, tosylate or mesylate and P is hydrogen, tetrahydropyranyl, benzyl or acetyl. Compounds of Formulae (XIX) and (XX) may be reacted together in a solvent such as dimethyl formamide, acetone or aromatic hydrocarbon in the presence of a base for example sodium hydride or sodium or potassium carbonate.

Compounds of Formula (XI) may be made with, for example, vinyl-bis-(cyclopentadienyl)zirconium chloride in THF in the presence of a palladium (O) catalyst.

Compounds of Formula I may be used to control arthropod pests such as insects and acarines. Acaricidal activity has been found to be enhanced when $R^1$ is hydrogen and $R^2$ has an alkyl substituent $\alpha$ to the nitrogen, for example when $R^2$ is 1,2-dimethylpropyl.

The compounds of Formula (I) may be used for such purposes by application of the compounds themselves or in diluted form in known fashion as a dip, spray, lacquer, foam, dust, powder, aqueous suspension, paste, gel, shampoo, grease, combustible solid, vapour emanator (e.g. coil, mat or the like), granule, aerosol, oil suspensions, oil solutions, pressure-pack, impregnated article (such as a plastics ear tag or collar or a strip to treat the air of an enclosed space) or pour on formulation. Dip concentrates are not applied per se, but diluted with water and the animals immersed in a dipping bath containing the dip wash. Sprays may be applied by hand or by means of a spray race or arch. The animal, plant or surface may be saturated with the spray by means of high volume application or superficially coated with the spray by means of light or ultra low volume application. Aqueous suspensions may be applied to the animal in the same manner as sprays or dips. Dusts may be distributed over the animals by means of a powder applicator or incorporated in perforated bags attached to trees or rubbing bars. Pastes, shampoos and greases may be applied manually or distributed over the surface of an inert material against which animals rub and transfer the material to their skins. Pour-on formulations are dispensed as a unit of liquid of small volume on to the backs of animals such that all or most of the liquid is retained on the animals.

The compounds of Formula (I) may be formulated either as formulations ready for use on the animals or as formulations requiring dilution prior to application, but both types of formulation comprise a compound of Formula (I) in intimate admixture with one or more carriers or diluents. The carriers may be liquid, solid or gaseous or comprise mixtures of such substances, and the compound of Formula (I) may be present in a concentration of from 0.025 to 99% w/v depending upon whether the formulation requires further dilution.

Dusts, powder and granules comprise the compound of Formula (I) in intimate admixture with a powdered solid inert carrier for example suitable clays, kaolin,

talc, mica, chalk, gypsum, vegetable carriers, starch and diatomaceous earths.

Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil), a wettable powder or a controlled release formulation, such as a microencapsulated formulation. The concentrate preferably comprises a mixture of the active ingredient, with or without an organic solvent and one or more emulsifiers. Solvents may be present within wide limits but preferably in an amount of from 0 to 90% w/v of the composition and may be selected from kerosene, ketones, alcohols, xylene, aromatic naphtha, and other solvents known in the formulating art. The concentration of emulsifiers may be varied within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, and other solvents known in the art.

Wettable powders and emulsifiable concentrates will normally contain from 5 to 95% by weight of the active ingredient, and are diluted, for example with water, before use. Microencapsulated formulations may be made by any known technique, for example coacervation or inter-facial polymerisation.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of formula (I) in intimate admixture with a dispersing agent and one or more surface active agents.

Aqueous suspensions of a compound of formula (I) may comprise a suspension in water together with suspending, stabilizing or other agents. Aqueous solutions may also be formed from acid addition salts of a compound of the formula (I). The suspensions or solutions may be applied per se or in a diluted form in known fashion. Electrostatic spraying techniques may be used with suitable formulations.

Greases (or ointments) may be prepared from vegetable oils, synthetic esters of fatty acids or wool fat together with an inert base such as soft paraffin. A compound of formula (I) is preferably distributed uniformly through the mixture in solution or suspension. Greases may also be made from emulsifiable concentrates by diluting then with an ointment base.

Pastes and shampoos are also semi-solid preparations in which a compound of formula (I) may be present as an uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of formula (I) required for treatment.

Aerosol sprays may be prepared as a simple solution of the active ingredient in the aerosol propellant and co-solvent such as halogenated alkanes and the solvents referred to above, respectively. Pour-on formulations may be made as a solution or suspension of a compound of formula (I) in a liquid medium which also contains a viscous oil to minimise spreading of the formulation on the surface of the animals. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body.

The concentration of the compound of Formula (I) to be applied to a locus (e.g. animal, grain, crop, soil, building etc.) will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the

formulation but in general will lie in the range of from 0.0001% to 0.5% except for undiluted formulations such as pour-on formulations which in general will be deposited at a concentration in the range from 0.1 to 20.0% and preferably 0.1 to 10%. For public health usage, a deposited concentration of up to about 5% may be needed. The concentrate may contain up to 90% active ingredient.

Dusts, greases, pastes and aerosol formulations are usually applied in a random fashion as described above and concentrations of 0.001 to 20% w/v of a compound of Formula (I) in the applied formulation may be used.

Bait formulations for, for example, cockroaches will include suitable attractants and/or foodstuffs. The compounds of the invention can be formulated specifically for use on grain or on the exposed surfaces of buildings, or for space spraying.

The compounds may be administered in an animal's feed to combat insect larvae infesting the animal's dung. Any suitable formulation, including microencapsulated material, may be used. The amount of the compound which is administered will vary according to the type and size of animal, and is chosen to provide a suitable concentration of the compounds in the animal's dung. Typically, 0.001 to 100 mg/kg bodyweight, preferably 0.1 to 10 mg/kg, are administered daily, to give concentrations of 0.001 to 1%, preferably 0.01 to 0.1% compound in the dung. The compound will usually be formulated as a concentrate or premix for mixing with a feed supplement, feed concentrate, roughage or the like. Alternatively, the compound may be added to the supply of drinking water. Suitable animals include cattle, pigs, horses, sheep, goats and poultry.

Insect pests include members of the orders Coleoptera (e.g. Anobium, Tribolium, Sitophilus, Diabrotica, Anthonomus, Hylotrupes or Anthrenus spp.), Lepidoptera (e.g. Ephestia, Plutella, Chilo, Heliothis, Spodoptera, Tinea or Tineola spp.), Diptera (e.g. Anopheles, Simulium, Musca, Aedes, Culex, Glossina, Stomoxys, Haematobia, Tabanus, Hydrotaea, Lucilia, Chrysomia, Callitroga, Dermatobia, Hypoderma, Liriomyza, and Melophagus spp.), Phthiraptera (Malophaga e.g. Damalina spp. and Anoplura e.g. Linognathus and Haematopinus spp.), Hemiptera (e.g. Triatoma, Rhodnius, Aphis, Bemisia, Aleurodes, Nilopavata, Nephrotetix or Cimex spp.), Orthoptera (e.g. Schistocerca or Acheta spp.), Dictyoptera (e.g. Blattella, Periplaneta or Blatta spp.), Hymenoptera (e.g. Solenopsis or Monomorium spp.), Isoptera (e.g.

Reticulitermes spp.)] Siphonaptera (e.g. Ctenocephalides or Pulex spp.), Thysanura (e.g. Lepisma spp.), Dermaptera (e.g. Forficula spp.) and Psocoptera (e.g. Peripsocus spp.). Acarine pests include ticks, e.g. members of the genera Boophilus, Rhipicephalus, Amblyomma, Hyalomma, Ixodes, Haemaphysalis, Dermocentor and Anocentor, and mites and manges such as Tetranychus, Psoroptes, Psorergates, Chorioptes, Demodex, Dermatophagoides, Acarus, Tyrophagus and Glycyphagus Spp.

The compounds exhibit killing and/or knockdown activity against adult and/or larval arthropod pests.

Compounds of the invention may be combined with one or more other active ingredients (for example pyrethroids, carbamates and organophosphates) and/or with attractants and the like. Furthermore, it has been found that the activity of the compounds of the invention may be enhanced by the addition of a synergist or potentiator, for example: one of the oxidase inhibitor class of synergists, such as piperonyl butoxide or NIA 16388; a second compound of the invention; or a pyrethroid pesticidal compound. When an oxidase inhibitor synergist is present in a formula of the invention, the ratio of synergist to compound of Formula (I) will be in the range 25:1-1:25 eg about 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin).

It will be understood that what we will claim may comprise:

(a) compounds of Formula (I);
(b) processes for the preparation of compounds of Formula (I);
(c) insecticidal and acaricidal compositions comprising a compound of Formula (I) in admixture with a carrier;
(d) processes for the preparation of such pesticidal compositions;
(e) methods for the control of insect or acarine pests comprising the application to the pest or its environment of a compound of Formula (I);
(f) synergised pesticidal compositions comprising a compound of Formula (I); and
(g) potentiating or non-potentiating mixtures of a compound of Formula (I) and another pesticidal compound;

(h) novel intermediates of the preparation of compounds of Formula (I), particularly compounds of Formulae (II), (A) or (B).

The following Examples illustrate, in a non-limiting manner, preferred aspects of the invention. All temperatures are in degrees Celsius.

Example 1: (2E,4E) N-Isobutyl 11-(1'-naphthyloxy)-2,4-undecadienamide

1-Naphthol (5g) was added to sodium hydride (1.7g of 50% dispersion) in dry dimethylformamide (200ml). The mixture was stirred for 20 mins. at room temperature under nitrogen and treated with 7-bromo heptan-1-ol (5.4g). After 24hrs. at room temperature the reaction was worked up in standard fashion and the crude material purified to give 7-(1'-naphthyloxy)-heptan-1-ol (3.6g).

The above alcohol (3.6g) in dichloromethane (20ml) was reacted at -60°, under nitrogen, with oxalyl chloride (2.63g) and dimethyl sulphoxide (3.3g) in dichloro methane. After treatment with triethylamine (20ml), warming to room temperature and standard work up, 7-(1'-naphthyloxy)-heptan-1-al (3.5g) was obtained.

Triethylphosphonocrotonate (3.55g) in tetrahydrofuran (THF) (25ml) was added at -70°, under nitrogen, to lithium diisopropylamide (14.4 mmol) in dry THF (50ml). The temperature of the mixture was allowed to reach -10°, recooled to -40° and 7-(1'-naphthyloxy)-heptan-1-al (3.64g) in THF was added. After 18hrs. at room temperature the reaction mixture was worked up in the standard fashion and the crude product purified by column chromatography to give (2E,4E) ethyl 11-(1'-naphthyloxy)-2,4-undecadienoate (2.94g).

The above ester was subjected to hydrolysis by aqueous potassium hydroxide in ethanol to give (2E,4E) 11-(1'-naphthyloxy)-2,4-undecanoic acid (1.9g), (m.p. 99-100°C; $R_f$ 0.23 on silica, 8:2 ether:hexane).

The above acid (0.4g) and triethylamine (175µl) in dichloromethane were treated with phenyl N-phenylphosphoramidochloridate (0.33g). After 2 hrs. the solution was treated with triethylamine (125µl) and then isobutylamine (175µl). After 24hrs. the reaction was worked up and the crude product purified by chromatography to give (2E,4E) N-isobutyl 11-(1'-naphthyloxy)-2,4-dienamide (0.3g) as a colourless solid. m.p. 75-7° Tlc. Silica-hexane/ether (1:1), 1 spot $R_f$ 0.27.

RSB/OLM/26th August 1986

NMR: 6.73-8.2 (8H), m,aryl,H3; 6.06(2H),m,H4,H5; 5.70(1H),d,H2; 5.60 (1H), NH: 4.16 (2H), t, H11; 2.18 (2H),m, H6; 1.20-2.00 (8H),m,H7,8,9,10; 3.18(2H,d of d; 1.50 (1H),m; 0.95(6H),d;isobutyl.

By using analogous methods and the starting materials given below, the compounds listed in Table 1 were made:

Table 1

$ArO(CH_2)_m(CH{=}CH)_2CONH^1R^2$

| Example No. | Ar | m | $R^2$ | m.p. | $R_f$ |
|---|---|---|---|---|---|
| 2 | 1-Naphthyl | 4 | isobutyl | | 0.21 |
| 3 | 1-Naphthyl | 4 | 1,2-dimethylpropyl | 100-101 • | |
| 4 | 1-Naphthyl | 5 | isobutyl | 137.5 | |
| 5 | 1-Naphthyl | 5 | 1,2-dimethylpropyl | | 0.29 |
| 6 | 1-Naphthyl | 6 | 1,2-dimethylpropyl | | 0.31 |
| 7 | 1-Naphthyl | 7 | isobutyl | | 0.29 |
| 8 | 1-Naphthyl | 7 | 1,2-dimethylpropyl | | 0.32 |
| 9 | 4-chloro-1-naphthyl | 4 | isobutyl | 109-13 | |
| 10 | 4-chloro-1-naphthyl | 4 | 1,2-dimethylpropyl | 89-91 | |
| 11 | 4-chloro-1-naphthyl | 6 | isobutyl | | 0.49 |
| 12 | 4-chloro-1-naphthyl | 6 | 1,2-dimethylpropyl | | 0.51 |
| 13 | 2,4-dichloro-1-naphthyl | 4 | isobutyl | | 0.39 |
| 14 | 2,4-dichloro-1-naphthyl | 4 | 1,2-dimethylpropyl | | 0.46 |
| 15 | 2,4-dichloro-1-naphthyl | 6 | isobutyl | | 0.49 |
| 16 | 2,4-dichloro-1-naphthyl | 6 | 1,2-dimethylpropyl | | 0.49 |
| 17 | 7-chloro-1-naphthyl | 4 | 1,2-dimethylpropyl | | 0.36 |
| 18 | 7-chloro-1-naphthyl | 4 | isobutyl | | 0.35 |
| 19 | 5-chloro-1-naphthyl | 4 | 1,2-dimethylpropyl | | 0.36 |
| 20 | 5-chloro-1-naphthyl | 4 | isobutyl | | 0.35 |
| 21 | 5-chloro-1-naphthyl | 4 | 2,2-dimethylpropyl | | 0.41 |
| 22 | 7-chloro-1-naphthyl | 4 | 2,2-dimethylpropyl | | 0.46 |
| 23 | 7-fluoro-1-naphthyl | 4 | 1,2-dimethylpropyl | 120-2 | |
| 24 | 7-fluoro-1-naphthyl | 4 | isobutyl | 111 | |
| 25 | 7-bromo-1-naphthyl | 4 | 1,2-dimethylpropyl | | 0.19 |

| Example No. | Ar | m | $R^2$ | m.p. | $R_f$ |
|---|---|---|---|---|---|
| 26 | 5,6,7,8-tetrafluoro-1-naphthyl | 4 | 1,2-dimethylpropyl | 151-2 | |
| 27 | 1-naphthyl | 4 | 2,2-dimethylpropyl | 77-8 | |
| 28 | 6-chloro-1-naphthyl | 4 | 1,2-dimethylpropyl | 112-3 | |
| 29 | 6 and 7-trifluoromethyl-1-naphthyl (mixture of regioisomers) | 4 | 1,2-dimethylpropyl | 104-6 | |
| 30 | 1-naphthyl | 4 | cyclohexyl | 127-8 • | |
| 31 | 1-naphthyl | 4 | 2-methyl-prop-2-enyl | 78-80 • | |
| 32 | 1-naphthyl | 4 | 2-(1,3-dioxalan-2-yl) propyl | | 0.15 |

t.l.c. was on silica with ether: hexane 2:8 (Ex No's. 2 to 8), 10:0 (Ex. No's. 11-22) or 1:1 (Ex. No's 25-29)

| Starting Material | Source/Reference |
|---|---|
| 4-chloro-1-naphthol | Aldrich |
| 2,4-dichloro-1-naphthol | Aldrich |
| 7-chloro-1-naphthol | A.M.El-Abbady et al J. Org. Chem., 1961, 26, 4871 |
| 5-chloro-1-naphthol | A.P. Lurie et al JACS, 1961, 83, 5015 |
| 7-fluoro-1-naphthol | W. Adcock JACS, 1967, 89, 386 |
| 7-bromo-1-naphthol | R.C. Fuson et al JACS, 1925, 47, 516 |
| 5,6,7,8-tetrafluoro-1-naphthol | G.W. Gribble et al J. Org. Chem., 1985, 50, 1614 |
| 6-chloro-1-naphthol | H. Erdmann Justus Liebigs Ann. Chem., 1888, 247, 372 |
| 6- & 7-$CF_3$-1-naphthol | P. Seiler et al Tet.Lett., 1971(20), 1683-6 |

## Biological Activity

### A. Topical application versus house flies

Compounds of the invention were administered topically in Cellosolve solution, with or without 6 µg piperonyl butoxide, to adult female Musca domestica (WRL strain). The compounds of Examples 2,3,7,8,10,12-15,18,19,21,22 and 25 to 28 were active at a dose of 10µg or less when unsynergised, and the compounds of Examples 1 to 29 were all active at 6µg or less when synergised.

### B. Biological Activity versus ticks

In injection into adult female Boophilus microplus, the compounds of Examples 3,6,8,9,17,19,23 and 26 to 29 were active at inhibiting reproduction and/or killing the ticks at 10 µg per tick. In an immersion test, the compound of Example 3 inhibited reproduction of 50% of B.microplus females at a concentration of 0.006%.

### C. Spray tests

The compounds of Examples 28 and 30 to 32 were sprayed as aqueous emulsions onto Musca domestica, Plutella xylostella and Tetranychus urticae. The $EC_{50}$'s (effective concentration for death of half the pests, as ppm) were as follows:

| Compound of Ex. No. | M.d | P.x. | T.u. |
|---|---|---|---|
| 28 | <200 | c1000 | c1000 |
| 30 | <1000 | >1000 | >1000 |
| 31 | <200 | c1000 | <1000 |
| 32 | <200 | <1000 | <1000 |

### Comparative Examples

Compounds were tested by the method of Example A above, using a dose of 3µg, with or without 6µg of piperonyl butoxide.

| Compound | Synergist | % Knockdown at 10 min | 1 hour | % Kill at 1 day | 2 days |
|---|---|---|---|---|---|
| Example 3 | - | 10 | 100 | 60 | 40 |
| | + | 10 | 100 | 100 | 100 |
| 2-naphthyloxy | - | 0 | 13 | 7.5 | 15 |
| analogue of Ex.3 | + | 0 | 0 | 0 | 5 |
| Example 2 | - | 90 | 100 | 100 | 100 |
| | + | 50 | 100 | 80 | 80 |
| 2-naphthyloxy | - | 0 | 0 | 7.5 | 7.5 |
| analogue of Ex. 2 | + | 0 | 0 | 0 | 5 |

RSB/OLM/26th August 1986

## Formulations

1. Emulsifiable Concentrate

| | |
|---|---|
| Compound of Example 1 | 10.00 |
| Ethylan KEO | 20.00 |
| Xylene | 67.50 |
| Butylated Hydroxyanisole | 2.50 |
| | 100.00 |

2. Wettable Powder

| | |
|---|---|
| Compound of Example 1 | 25.0 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

3. Dust

| | |
|---|---|
| Compound of Example 1 | 0.50 |
| Butylated Hydroxyanisole | 0.10 |
| Talc | 99.40 |
| | 100.00 |

4. Bait

| | |
|---|---|
| Compound of Example 1 | 40.25 |
| Icing Sugar | 59.65 |
| Butylated hydroxy toluene | 0.10 |
| | 100.00 |

5. Lacquer

| | |
|---|---|
| Compound of Example 1 | 2.5 |
| Resin | 5.0 |
| Butylated Hydroxy anisole | 0.5 |
| High aromatic white spirit | 92.0 |
| | 100.00 |

6. Aerosol

| | |
|---|---|
| Compound of Example 1 | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

7. Spray

| | |
|---|---|
| Compound of Example 1 | 0.1 |
| Butylated Hydroxy anisole | 0.1 |
| Xylene | 10.0 |
| Odourless Kerosene | 89.8 |
| | 100.00 |

8. Potentiated Spray

| | |
|---|---|
| Compound of Example 1 | 0.1 |
| Permethrin | 0.5 |
| Butylated Hydroxyanisole | 0.1 |
| Xylene | 10.1 |
| Odourless Kerosene | 89.2 |
| | 100.0 |

Claims

1. A compound of Formula (I):

(I) Ar-O-$(CH_2)_m$ $(CH{=}CH)_a$ $CONR^1R^2$

wherein Ar is a group 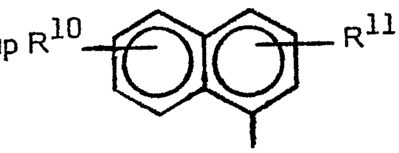

in which each of $R^{10}$ and $R^{11}$ is independently one or more of hydrogen, $C_{1-6}$ alkyl, halo-$C_{1-6}$ alkyl, halo, $C_{1-6}$ alkoxy or halo $C_{1-6}$ alkoxy

a = 1 or 2
m = 1 to 14

and $R^1$ and $R^2$ are independently selected from hydrogen, $C_{1-6}$ alkyl and $C_{3-6}$ cycloalkyl (either of which may be substituted by halo, $C_{2-6}$ alkenyl, $C_{1-6}$ alkyl $C_{3-6}$ cycloalkyl, a $C_{3-6}$ heterocycle wherein the heteroatoms are one or more oxygen atoms, $C_{1-6}$ alkoxy or $C_{2-6}$ alkynyl).

2. A compound according to claim 1 wherein a is 2 and the double bonds conjugated to the amide carbonyl are in the E configuration..

3. A compound according to claim 1 or 2 wherein m is an even number.

4. A compound according to claim 3 wherein m is 4 or 6.

5. A compound according to any one of the preceding claims wherein $R^1$ is hydrogen and $R^2$ is isobutyl, 1,2-dimethylpropyl or 2,2-dimethylpropyl.

6. A compound according to any one of the preceding claims wherein one of $R^{10}$ and $R^{11}$ is hydrogen and the other is hydrogen, halo or trifluorometh yl.

7. A process for preparing a compound according to claim 1
   (a) by amidation of the corresponding acid or acid derivative, ie by reaction of a compound of Formula (II) with a compound of Formula (III) or derivative thereof

(II) $Ar\ O(CH_2)_m\ (CH{=}CH)_a CO\ Z^1$ (III) $HNR^1R^2$

wherein $Z^1$ is hydroxyl, halo, or a phosphoroimidate ester group (-P(→0)(OAryl) NH aryl) and the other variables are as defined in claim 1;

(b) by reaction of a compound of formula (IV) with a compound of formula (V) or (VI)

(IV) $ArO(CH_2)_m(CH{=}CH)_pCHO$

(V) $(Z'')_3P{=}CH\ (CH{=}CH)_q\ C(0)NR^1R^2$

(VI) $(Z'')_2P(0){=}CH(CH{=}CH)_qC(0)NR^1R^2$

wherein Z" is alkyl, alkoxy or aryl, one of p and q is 0 and the other is 0 or 1;

(c) by oxidation and elimination reactions on a compound of Formula (VII) or (VIII):

(VII) $Ar\text{-}O\text{-}(CH_2)_m(CH{=}CH)_{a-1}\ C(X)H\ C(Y)HC(O)NR^1R^2$

(VIII) $Ar\text{-}O\text{-}(CH_2)_mC(X)HC(Y)HCH(CH{=}CH)_{a-1}\ C(O)NR^1R^2$

wherein one of X and Y is hydrogen and the other is a group Q(→O)L, Q is sulphur or selenium and L is lower alkyl or aryl;

(d) by reaction of a compound of Formula (IX) with a compound of Formula (X):-

(IX) $Ar\text{-}O(CH_2)_mCH_2\text{-}Hal$

(X) $HC{\equiv}CH(CH{=}CH)_{a-1}C(O)NR^1R^2$

where Hal is a halogen atom, followed by reduction of the triple bond;

(e) by reacting a compound of Formula (XI) with a compound of Formula (XII):

(XI) $Ar\text{-}O(CH_2)_m\ CH{=}CH\text{-}M$

(XII) $Hal\text{-}(CH{=}CH)_{a-1}C(O)NR^1R^2$

wherein Hal is halide and M is a metal atom or metal group; or

(f) by reduction of the triple bond in a compound of Formula (A) or (B):

(A) $Ar\,O\,(CH_2)_m\,C{\equiv}C\text{-}\,(CH{=}CH)_{a-1}\text{-}\,CO\,NR^1R^2$

(B) $Ar\,O\,(CH_2)_m\,(CH{=}CH)_{a-1}\text{-}\,C{\equiv}C\text{-}\,CO\,NR^1R^2$

8. A pesticidal composition comprising a compound according to any one of claims 1 to 6 and one or more diluents, carriers or the like.

9. A method of combatting pests by applying to a pest or its locus a compound according to any one of claims 1 to 6 or a composition according to claim 8.

10. A compound of Formula (II):

(II) $Ar\,O\,(CH_2)_m\,(CH{=}CH)_a\,COZ^1$

wherein Ar, m and a are as defined in claim 1 and $Z^1$ is hydroxyl, halo or a phosphoro imidate ester group.

RSB/OLM/26th August 1986

Claims

1. A process for preparing a compound of Formula (I):

(I) $Ar\text{-}O\text{-}(CH_2)_m\ (CH{=}CH)_a\ CONR^1R^2$

wherein Ar is a group 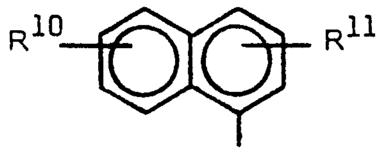

in which each of $R^{10}$ and $R^{11}$ is independently one or more of hydrogen, $C_{1-6}$ alkyl, halo-$C_{1-6}$ alkyl, halo, $C_{1-6}$ alkoxy or halo $C_{1-6}$ alkoxy

a = 1 or 2
m = 1 to 14

and $R^1$ and $R^2$ are independently selected from hydrogen, $C_{1-6}$ alkyl and $C_{3-6}$ cycloalkyl (either of which may be substituted by halo, $C_{2-6}$ alkenyl, $C_{1-6}$ alkyl $C_{3-6}$ cycloalkyl, a $C_{3-6}$ heterocycle wherein the heteroatoms are one or more oxygen atoms, $C_{1-6}$ alkoxy or $C_{2-6}$ alkynyl),

(a) by amidation of the corresponding acid or acid derivative, ie by reaction of a compound of Formula (II) with a compound of Formula (III) or derivative thereof(II)Ar $O(CH_2)_m\ (CH{=}CH)_aCO\ Z^1$ (III) $HNR^1R^2$

wherein $Z^1$ is hydroxyl, halo, or a phosphoroimidate ester group (-P(→O)(OAryl) NH aryl) and the other variables are as defined in claim 1;

(b) by reaction of a compound of formula (IV) with a compound of formula (V) or (VI)

(IV) $ArO(CH_2)_m(CH{=}CH)_pCHO$
(V) $(Z")_3P{=}CH\ (CH{=}CH)_q\ C(O)NR^1R^2$

(VI) $(Z")_2P(O){=}CH(CH{=}CH)_qC(O)NR^1R^2$

wherein Z" is alkyl, alkoxy or aryl, one of p and q is 0 and the other is 0 or 1;

(c) by oxidation and elimination reactions on a compound of Formula (VII) or (VIII):

(VII) $Ar\text{-}O\text{-}(CH_2)_m(CH{=}CH)_{a-1}\ C(X)H\ C(Y)HC(O)NR^1R^2$

(VIII) $Ar\text{-}O\text{-}(CH_2)_mC(X)HC(Y)HCH(CH{=}CH)_{a-1}\ C(O)NR^1R^2$

wherein one of X and Y is hydrogen and the other is a group Q(→O)L, Q is sulphur or selenium and L is lower alkyl or aryl;

(d) by reaction of a compound of Formula (IX) with a compound of Formula (X):-

(IX) $Ar\text{-}O(CH_2)_mCH_2\text{-}Hal$

(X) $HC{\equiv}CH(CH{=}CH)_{a-1}C(O)NR^1R^2$

where Hal is a halogen atom, followed by reduction of the triple bond;

(e) by reacting a compound of Formula (XI) with a compound of Formula (XII):

(XI) $Ar\text{-}O(CH_2)_m\ CH{=}CH\text{-}M$

(XII) $Hal\text{-}(CH{=}CH)_{a-1}C(O)NR^1R^2$

wherein Hal is halide and M is a metal atom or metal group; or

(f) by reduction of the triple bond in a compound of Formula (A) or (B):

(A) $Ar\ O\ (CH_2)_m\ C{\equiv}C\text{-}\ (CH{=}CH)_{a-1}\text{-}\ CO\ NR^1R^2$

(B) $Ar\ O\ (CH_2)_m\ (CH{=}CH)_{a-1}\text{-}\ C{\equiv}C\text{-}\ CO\ NR^1R^2$

2. A process according to claim 1 wherein a is 2 and the double bonds conjugated to the amide carbonyl are in the E configuration.

3. A process according to claim 1 or 2 wherein m is an even number.

RSB/OLM/27th August 1986

4. A process according to claim 3 wherein m is 4 or 6.

5. A process according to any one of the preceding claims wherein $R^1$ is hydrogen and $R^2$ is isobutyl, 1,2-dimethylpropyl or 2,2-dimethylpropyl.

6. A process according to any one of the preceding claims wherein one of $R^{10}$ and $R^{11}$ is hydrogen and the other is hydrogen, halo or trifluoromethyl.

7. A pesticidal composition comprising a compound prepared according to any one of claims 1 to 6 and one or more diluents, carriers or the like.

8. A method of combatting pests by applying to a pest or its locus a compound prepared according to any one of claims 1 to 6 or a composition according to claim 7.

RSB/OLM/27th August 1986

0225011

Application number

European Patent Office

# EUROPEAN SEARCH REPORT

EP 86307436.5

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl 4) |
| D,A | EP - A2 - 0 143 593 (NATIONAL RESEARCH DEVELOPMENT CORPORATION)<br>* Claims; table IV *<br>-- | 1,7-9 | C 07 C 103/58<br>C 07 C 102/00<br>C 07 C 59/68<br>C 07 D 317/28<br>C 07 F 9/44<br>A 01 N 39/02<br>A 01 N 43/00 |
| A | EP - A1 - 0 109 311 (STAUFFER CHEMICAL COMPANY)<br>* Pages 4-6; claims *<br>---- | 1,7-10 | |
| | | | TECHNICAL FIELDS SEARCHED (Int. Cl 4) |
| | | | C 07 C 103/00<br>C 07 C 59/00<br>C 07 F<br>A 01 N |

The present search report has been drawn up for all claims

| Place of search | Date of completion of the search | Examiner |
|---|---|---|
| VIENNA | 18-12-1986 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82